# EUROPÄISCHE PATENTANMELDUNG

(11) **EP 3 964 758 A1**
(43) Veröffentlichungstag der Anmeldung: **09.03.2022**
(21) Anmeldenummer: 21195604.0
(22) Anmeldetag: 08.09.2021
(51) Int. Cl.: F24F 8/10, F24F 8/22, F24F 8/80, F24F 13/20, F24F 11/39, F24F 11/52, F24F 8/26, F24F 13/065, A61L 9/20, F24F 13/06, F24F 7/007, F24F 13/28, F24F 110/66, F24F 110/70

(54) **LUFTUMWÄLZUNGSGERÄT ZUR REINIGUNG DER LUFT VON POTENTIELL MIT VIREN BELASTETEN AEROSOLEN**

(30) Priorität: 08.09.2020 DE 102020123397
(71) Anmelder: Mel-Lop AG, 85386 Eching (DE)
(72) Erfinder: Langer, Hans-J., 85386 Eching (DE)
(74) Vertreter: Misselhorn, Hein-Martin

(57) **Zusammenfassung**

Für Versammlungsräume wird eine leicht nachträglich aufzustellende Lüftung konzipiert, die sicher vor virenbelasteten Aerosolen schützen soll. Dabei wird von der Anlage im Versammlungsraum ein vertikaler Luftstrom generiert, der verhindern soll, dass die virenhaltige Ausatemluft von einer Person auf die nächste Person übertragen wird. Hierzu wird von der Lüftungssäule oben die desinfizierte Luft in den Raum geblasen und sinkt an den Menschen vertikal nach unten, um dort wieder von der Lüftung eingesaugt zu werden. In der Lüftungssäule wird die Luft von einem Ventilator eingesaugt und über einen mechanischen Filter geblasen, der gröbere Teile abscheidet, anschließend wird die Luft von UV-C Lampen desinfiziert und tritt oben über eine flache Düse rund herum in den Raum. Der Luftstrom ist stark genug, um bis zu 5 m horizontal an der Decke in den Raum zu blasen.

## Beschreibung

Die Erfindung betriff ein Luftumwälzungsgerät zur Reinigung der Luft von potentiell mit Viren belasteten Aerosolen nach dem Oberbegriff des Anspruchs 1.

Im Zeitalter der Corona-Pandemie besteht verstärkter Bedarf an Luftumwälz- bzw. -reinigungsgeräten, die dazu beitragen können, die Ansteckungsgefahr zu verringern.

### Technischer Hintergrund

Die bekannten Lüftungen bzw. Luftreiniger für den Innenraum leiden an dem Problem, dass sie Luftströmungen verursachen, die gerade auch im Kopfhöhenbereich der im Raum befindlichen Personen eine starke horizontale Geschwindigkeitskomponente aufweisen.

Das ist problematisch, denn hierdurch wird das von einer Person ausgeatmete Aerosol durch die von dem Luftreiniger verursachte Strömung unter Umständen auf kürzestem Wege in den Einatembereich von dessen Nachbarn getragen.

### Aufgabe der Erfindung

Es ist die Aufgabe der Erfindung, einen Luftreiniger zu schaffen, der einfach aufgebaut ist, sehr effizient arbeitet und die Gefahr verringert, dass von einer Person ausgeatmetes Aerosol durch die Strömung auf kürzestem Wege in den Einatembereich von dessen Nachbarn getragen wird, ohne zuvor den Luftreiniger durchlaufen zu haben.

### Erfindungsgemäße Lösung

Die erfindungsgemäße Lösung erfolgt mit den Mitteln des Anspruchs 1.

Sie zeichnet sich dadurch aus, dass die von dem Luftumwälzungsgerät angesaugte Luft nicht nur gefiltert und bestrahlt wird, sondern dass sie innerhalb des Luftumwälzungsgeräts bis in den Überkopfbereich der sich bestimmungsgemäß in dem Raum aufhaltenden Personen geführt wird, um erst dort wieder nach außen in den Raum abgegeben zu werden. Dadurch ist es bei richtiger Geräteauslegung so, dass sich die von dem Luftumwälzungsgerät abgegebene Luft zunächst, solange ihre horizontale Geschwindigkeitskomponente groß ist, oberhalb der im Raum befindlichen Personen in den Raum ausbreitet. Erst dann, wenn ihre horizontale Geschwindigkeitskomponente überwiegend abgebaut ist, sinkt die Luft bis in den Kopfbereich der im Raum befindlichen Personen ab, um dann dem Boden zuzustreben, um von dort aus in einer horizontalen Ansaugströmung dem Gerät zumindest teilweise wieder zugeführt zu werden.

Der wesentliche Unterschied dieser Vorrichtung liegt also in der Ausströmdüse und in der Raumhöhe zum Ausströmen und dem Lufteinlass am Boden, wodurch eine senkrechte Luftbewegung im Versammlungsraum erzielt wird und so eine Ansteckung von einer Person zur anderen vermieden wird, da die Ausatemluft jeder Person senkrecht nach unten abfließt und nicht zum Einatmen des Nachbarn horizontal weitergegeben wird. Bisherige Lüftungen für den Innenraum blasen eventuell direkt die ausgeatmeten Aerosole von einer Person zur anderen.

Einer der wichtigsten Anwendungsfälle der Erfindung sind Klassenzimmer.

Es gibt noch viele Klassenzimmer, die keine Lüftung mit einem notwendigen Luftwechsel für die kalte Jahreszeit haben. Hierzu wurde diese leicht aufstellbare Lüftung entwickelt, die nur passend in den Raum gestellt werden muss, um einen ansteckungsfreien Schulbetrieb ohne Mundschutzmaske zu erlauben. Wenn dazu noch Schutzscheiben auf dem Tisch zwischen den Schülern montiert werden, ist der Schutz im Schulzimmer komplett.

### Weitere optionale Ausgestaltungsmöglichkeiten der Erfindung

Für folgende weitere Merkmale wird allein, oder in Kombination mit einem der bereits aufgestellten Ansprüche, Schutz beansprucht. Man kann auch sagen, dass die nachfolgenden Merkmale bevorzugte Weiterentwicklungsmöglichkeiten der Erfindung sind.
- Bei richtiger Aufstellung bietet die Anlage eine Luftbewegung von 0,1 m/sec von oben nach unten. D.h. das kritische Aerosol ist nach 10 Sekunden oder zweimal Atmen um 1 m unter den Mundbereich abgesunken.
- Zum weiteren Schutz von Schülern an einem Schultisch werden Schutzscheiben als Trennung zwischen den Schülern empfohlen.
- Ein weiterer Unterschied zu den am Markt vorhandenen Lüftungen liegt in der Intensität der UV-C Strahlung und der Aufenthaltsdauer der vorbeiströmenden Luft in der UV-C Kammer. Durch die mechanische Vorfilterung wird sichergestellt, dass kein Aerosoltröpfchen mit Virus durch ein Staubpartikelchen vor dem UV-C Licht abgeschattet wird und so lebend die UV-C Zone verlässt.
- Ebenso ist die Aufenthaltsdauer und die Intensität der UV-C Strahlung so, dass 99,9 % aller virenhaltigen Aerosole virensicher abgetötet sind.
- Durch die spezielle Gestaltung der Ausströmdüse am Oberteil wird eine tiefe Ausblascharakteristik ohne Verzögerung durch Turbulenzen der Luft in den Raum erzielt.
- Da diese Anlage vor allem für Räume ohne Außenlüftung gedacht ist, kann die Anlage eine Ansteckung der Personen durch Viren in dem Aerosol verhindern, jedoch bietet die Anlage keine Frischluft mit Sauerstoff. Um zu verhindern, dass die Kohlenstoff-Konzentration zu groß wird, hat die Anlage einen CO2 Sensor mit Anzeige und einstellbarem Alarmniveau. Damit können die Fenster passend geöffnet werden.
- Eine weitere Sicherheit bietet ein UV-C Sensor, der Alarm gibt, falls die UV-C Lichtleistung ausfällt.
- Ebenso gibt es eine Überwachung des mechanischen Filters und des Lüftermotors, falls die Luftleistung absinkt.
- Um eine geringe Störung durch Lärm zu verursachen, wird eine Schalldämmung des Lüftermotors und der Zischgeräusche der Luftströmungen durchgeführt.
- Für eine Sterilisation der Oberflächen im Versammlungsraum gibt das System die Möglichkeit, für einen einstellbaren Zeitraum einen Ozongenerator einzuschalten. So können z.B. in der Nacht alle Oberflächen im Raum durch Ozon sterilisiert werden.
- Das Luftumwälzungsgerät kann so beschaffen sein, dass der Luftauslass (3) Lamellen hat, die die Luftströmungen so ausrichten, dass es einen gleichmäßigen Luftfluss über den umgebenden Raum gibt.
- Das Luftumwälzungsgerät kann so beschaffen sein, dass der CO2-Anteil der Raumluft überwacht und angezeigt wird.
- Das Luftumwälzungsgerät kann steckerfertig sein, d. h. so beschaffen sein, dass es an beliebiger freier Stelle ohne vorherige Montagearbeiten im Raum aufgestellt, und nach Anschluss an das Stromnetz mithilfe eines werkzeuglos zu bedienenden Steckers bestimmungsgemäß in Betrieb genommen werden kann.
- Das Luftumwälzungsgerät ist bevorzugt so beschaffen, dass es Luft aus dem Raum ansaugt, in dem es aufgestellt ist, und diese Luft auch wieder - bevorzugt vollständig - in diesen Raum abgibt.
- Das Luftumwälzungsgerät kann so beschaffen sein, dassnach einem der vorhergehenden Ansprüche, dadurch gekennzeichnet, dass das Luftumwälzungsgerät eine säulenartige Gestalt hat und aus einem Steigrohr besteht, das unterseitig auf einer horizontal über das Steigrohr hinausragenden Säulenbasis aufsteht und oberseitig von einem horizontal über das Steigrohr hinausragenden Säulenkapitell abgeschlossen wird, wobei die Luftansaugung über die Säulenbasis erfolgt und die Luftabgabe über das Säulenkapitell, wobei der Ventilator (4), der mechanische Luftfilter (5) und die UV-C Desinfektionseinheit vorzugsweise vollständig in dem Steigrohr untergebracht sind.
- Das Luftumwälzungsgerät kann so beschaffen sein, dass die von dem Luftumwälzungsgerät ausgestoßene Luft unmittelbar bei ihrem Austritt aus dem Gerät eine Geschwindigkeit von weniger als 0,1 m/s aufweist, idealerweise sogar von weniger als 0,05 m/s.
- Das Luftumwälzungsgerät kann so beschaffen sein, dass der durch die Düsen zur Wasserverneblung erzeugte Wassernebel eine Kühlung über der abzublasenden Fläche des Raumes bewirkt, wodurch die gewünschte Luftbewegung verstärkt wird, da die kältere Luft selbsttätig nach unten absinkt.
- Das Luftumwälzungsgerät kann so beschaffen sein, dass der durch die Düsen zur Wasserverneblung erzeugte Wassernebel eine Befeuchtung der Raumluft bewirkt, welche einen zusätzlichen Luftreinigungseffekt zur Folge hat, vor allem durch das Aufnehmen und Mitbefördern eventuell infizierter Aerosole durch die größeren Wassertröpfchen des Wassernebels.
- Das Luftumwälzungsgerät kann do beschaffen sein, dass das Sprühwasser, welches zur Wasservernebelung genutzt wird, desinfiziert wird, bevorzugt permanent und bevorzugt durch flüchtige Substanzen wie Ozon.
- Das Luftumwälzungsgerät kann bevorzugt so beschaffen sein, dass - bevorzugt an die Düsen des Luftauslasses (3) anschließend - Düsen zur Wasservernebelung angebracht sind.

### Ausführungsbeispiel

Fig 1:
   In einem mindestens 250 cm hohen Rohr 1 sind ein Lufteinlass 2 und ein Luftauslass 3 mit Ventilator 4 untergebracht. Der Ventilator 4 bläst die Luft über einen Filter 5 durch den Raum mit UV-C Lampen 6. Durch einstellbare Luftauslassdüsen 3 wird die geforderte Durchflutung 11 des Raumes mit desinfizierter Luft erzielt.
   Die Lichtstärke der UV-C Lampen im Wellenlängenbereich um 200 nm wird über einen Sensor 7 überwacht. Zur Überwachung des CO2 Gehalts der Luft ist ein CO2 Sensor 8 mit Anzeige vorhanden. Zur Desinfektion ist eine Ozon produzierende Röhre 9 eingebaut. Zur Überwachung des vom Ventilator erzeugten Druckes nach dem Filter ist ein Drucksensor 10 eingebaut. Gut zu erkennen ist auch die bevorzugt dem UV-VC-Lampenbereich strömungstechnisch vorgelagerte Heizung 15. Diese erhöht die Überkopfreichweite der Strömung, so dass ein größerer Raumbereich positiv beeinflusst werden kann. Zudem kann die zusätzliche Heizung ach deswegen sicherheitserhöhend sein, weil sich in der kalten Jahreszeit komfortabler lüften lässt.
Fig 2:
   Der Luftauslass 3 wird realisiert über konzentrische Rohre, so dass eine laminare Luftströmung bis zur Auslassdüse erzielt wird. Die Auslassdüsen 3 werden durch im Winkel einstellbare Klappen gebildet, so dass die Reichweite der Luftströmung 14 im Raum einstellbar ist.

### Bezugszeichenliste

- 1: Rohr
- 2: Lufteinlass
- 3: Luftauslass
- 4: Ventilator
- 5: Filter
- 6: UV-C Lampe
- 7: Sensor
- 8: Sensor
- 9: Ozon produzierende Röhre
- 10: Drucksensor
- 11: Durchflutung
- 12: Nicht vergeben in den Figuren
- 13: Nicht vergeben in den Figuren
- 14: Reichweite Luftströmung
- 15: Heizung

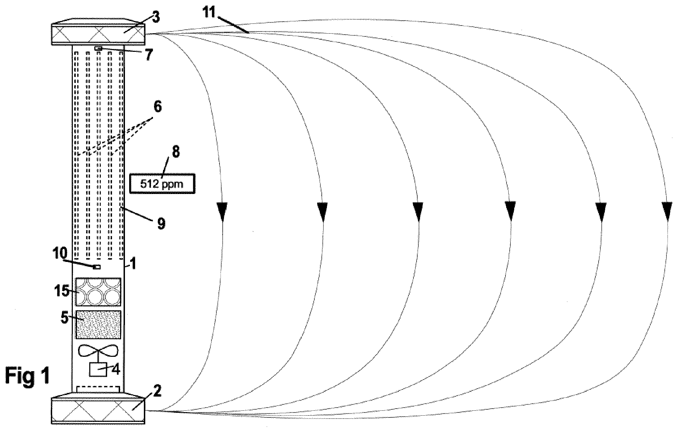

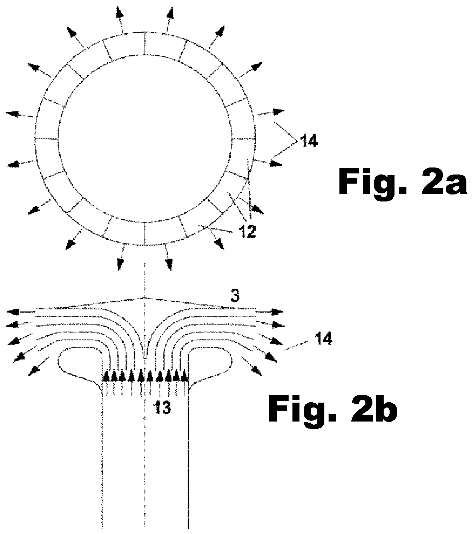

## Patentansprüche

1. Luftumwälzungsgerät zur Verringerung der Belastung von Raumluft mit infektiösem Aerosol, mit einem Ventilator (4), einem mechanischen Luftfilter (5) und einer UV-C Desinfektionseinheit zur Verringerung oder Beseitigung der Keimbelastung der durchströmenden Luft, **dadurch gekennzeichnet, dass** die Luftführung des Luftumwälzungsgeräts so gestaltet ist, dass nur in Bodennähe Luft angesaugt wird, die dann durch den Luftfilter (5) fließt und die UV-C Desinfektionseinheit passiert, wobei die Luft innerhalb des Luftumwälzungsgeräts bis in den Überkopfbereich der sich bestimmungsgemäß in dem Raum aufhaltenden Personen geführt wird, um dort nach außen in den Raum abgegeben zu werden, in dem das Luftumwälzungsgerät aufgestellt ist.

2. Luftumwälzungsgerät nach Anspruch 1, **dadurch gekennzeichnet, dass** die Luftführung so gestaltet ist, dass die Luft mindestens 2,5 m und idealerweise mindestens 3 m oberhalb der Ansaugöffnung des Luftumwälzungsgeräts nach außen in den Raum abgegeben wird, in dem das Luftumwälzungsgerät aufgestellt ist.

3. Luftumwälzungsgerät nach einem der vorhergehenden Ansprüche, **dadurch gekennzeichnet, dass** die Ansaugöffnung in Bodennähe ringförmig ist, so dass sie in horizontaler Richtung ansaugt und/oder, dass die Ausblasöffnung am oberen Ende des Luftumwälzungsgeräts ringförmig ist, so dass sie in horizontaler Richtung ausbläst.

4. Luftumwälzungsgerät nach einem der vorhergehenden Ansprüche, **dadurch gekennzeichnet, dass** die Luftführung und der Ventilator (4) so aufeinander abgestimmt sind, dass der von dem Luftumwälzungsgerät ausgestoßene Luftstrom mindestens 2 m, besser mindestens 2,5 m im Wesentlichen horizontal in den Raum ausströmt, bevor er den Bereich erreicht, in dem sich die Köpfe der bestimmungsgemäß in dem Raum befindlichen Personen befinden.

5. Luftumwälzungsgerät nach einem der vorhergehenden Ansprüche, **dadurch gekennzeichnet, dass** das Luftumwälzungsgerät einen Luftauslass (3) mit Ausströmdüsen besitzt und die Ausströmdüsen justierbar sind, um die vorgenannte Luftströmung zu erzielen.

6. Luftumwälzungsgerät nach einem der vorhergehenden Ansprüche, **dadurch gekennzeichnet, dass** die Lichtleistung der UV-C Leuchten (6) überwacht werden, derart, dass keine unbemerkte Leistungsverringerung eintreten kann.

7. Luftumwälzungsgerät nach einem der vorhergehenden Ansprüche, **dadurch gekennzeichnet, dass** das Luftumwälzungsgerät eine Alarmeinheit aufweist, die so beschaffen ist, dass sie Alarm gibt, wenn ein bevorzugt einstellbarer, bestimmter CO2-Anteil in der Luft überschritten wird.

8. Luftumwälzungsgerät nach einem der vorhergehenden Ansprüche, **dadurch gekennzeichnet, dass** das Luftumwälzungsgerät eine Einrichtung zur Einstellung der Geschwindigkeit des Luftstroms aufweist und eine Überwachungseinrichtung, die so beschaffen ist, dass sie Alarm gibt, wenn der Luftstrom eine bestimmte Geschwindigkeit unterschreitet, etwa wegen einer zunehmenden Verlegung des mechanischen Filters (5).

9. Luftumwälzungsgerät nach einem der vorhergehenden Ansprüche, **dadurch gekennzeichnet, dass** das Luftumwälzungsgerät mit einem Aerosolsensor (7) ausgerüstet ist, der so beschaffen ist, dass die Aerosolanteile der Luft angezeigt werden.

10. Luftumwälzungsgerät nach einem der vorhergehenden Ansprüche, **dadurch gekennzeichnet, dass** das Luftumwälzungsgerät mit einem Ozon-Generator ausgerüstet ist, der zur Raumdesinfektion Ozon abgibt und der bevorzugt mit einer Zeituhr gekoppelt ist, um die Ozonabgabe so zu steuern, dass sie zu Zeiten erfolgt, in denen der Raum bestimmungsgemäß nicht oder nur ausnahmsweise genutzt wird.

11. Luftumwälzungsgerät nach einem der vorhergehenden Ansprüche, **dadurch gekennzeichnet, dass** die Luftströmung vor den Auslassdüsen durch konzentrisch ineinander angeordnete Rohre oder Kanäle so geführt wird, dass sie laminar bleibt.

12. Luftumwälzungsgerät nach einem der vorhergehenden Ansprüche, **dadurch gekennzeichnet, dass** zwischen dem Ventilator (4) und dem Lufteintritt (2) in das Steigrohr ein sich vertikal erstreckender Strömungsberuhigungskanal angeordnet ist, der vorzugsweise mindestens 1/6, besser mindestens 1/4 der Länge des Steigrohrs ausmacht.

13. Luftumwälzungsgerät nach einem der vorhergehenden Ansprüche, **dadurch gekennzeichnet, dass** das Steigrohr eine UV-C-Kammer bildet, indem das Steigrohr an seiner Innenseite rundum mit UV-C-Leuchten (6), vorzugsweise in der Gestalt von UV-C-Dioden, besetzt ist, und die Länge dieser Kammer vorzugsweise mehr als 40 %, noch besser mehr als 50 % der Länge des Steigrohrs ausmacht.

14. Luftumwälzungsgerät nach einem der vorhergehenden Ansprüche, **dadurch gekennzeichnet, dass** zumindest das Steigrohr in einem es ummantelnden Gehäuse verläuft, wobei der Zwischenraum zwischen dem ummantelnden Gehäuse und dem Steigrohr bevorzugt mit schalldämmendem Material gefüllt ist.

15. Luftumwälzungsgerät nach einem der vorhergehenden Ansprüche, **dadurch gekennzeichnet, dass** das Luftumwälzungsgerät einen Luftkühler, vorzugsweise in Gestalt einer Klimaanlage, aufweist, so dass die über den Köpfen der im Raum befindlichen Personen in horizontaler Richtung abgegebene Kaltluft zu Boden sinkt, sobald ihre Geschwindigkeitskomponente in horizontaler Richtung soweit abgesunken ist, dass sie den Kopfbereich der im Raum befindlichen Personen erreicht und/oder, dass das Luftumwälzungsgerät einen Luftheizer aufweist, so dass die über den Köpfen der im Raum befindlichen Personen in horizontaler Richtung abgegebene Warmluft länger oben im Raum verbleibt und daher die horizontale Reichweite erhöht wird.
